# EUROPEAN PATENT APPLICATION

(11) **EP 3 622 985 A1**
(43) Date of publication of application: **18.03.2020**
(21) Application number: 17909446.1
(22) Date of filing: 21.07.2017
(51) Int. Cl.: A61M 5/20, A61M 5/315

(54) **INJECTION PEN**

(30) Priority: 08.05.2017 CN 201720503514 U
(71) Applicant: Gansu Changee Bio-pharmaceutical Co., Ltd., Tianshui, Gansu 741020 (CN)
(72) Inventor: GENG, Xusheng, Tianshui Gansu 741020 (CN); QU, Yanling, Tianshui Gansu 741020 (CN); HAN, Yitao, Tianshui Gansu 741020 (CN); PAN, Junfeng, Tianshui Gansu 741020 (CN); LI, Guoan, Tianshui Gansu 741020 (CN)
(74) Representative: Schollweck, Susanne
(86) International application number: PCT/CN2017/093813
(87) International publication number: WO 2018/205409

(57) **Abstract**

An injection pen, comprising a pen holder (6); the front end of the pen holder (6) is provided with a medicine bin (13); the rear end of the pen holder (6) is provided with a trigger (1); a dosage cylinder (3), a differential rod (5), and a transmission rod (10) are coaxially arranged in the pen holder (6) from outside to inside in sequence; the transmission rod (10) is connected with a key slot of the differential rod (5); the inner wall of the pen holder (6) is provided with a limiting protrusion matching the transmission rod (10); the front end of the dosage cylinder (3) is provided with a transmission clutch (8); the rear end of the transmission clutch (8) is provided with a latch; the transmission rod (10) is provided with an inner ring gear matching the latch; the transmission clutch (8) and the dosage cylinder (3) can be linked unidirectionally and transmittingly; the front end of the dosage cylinder (3) is further provided with a push rod base (9); the front portion of the push rod base (9) is provided with a push piece (11); a push rod (4) matching the push piece (11) is coaxially provided in the push rod base (9) in screw-thread fit fashion; and the push rod base (9) and the transmission rod (10) can be linked unidirectionally and transmittingly. The injection pen is easy to operate, and can improve the accuracy of dosage control.

## Description

The present application claims priority to Chinese Patent Application No.201720503514.8 titled "INJECTION PEN", filed with the China National Intellectual Property Administration on May 8, 2017, which is incorporated herein by reference in its entirety.

### FIELD

The present application relates to the technical field of medical apparatus and instruments, and in particular to an injection pen.

### BACKGROUND

The injection pen is a medical instrument widely used in clinical medical treatment for medication injection.

Currently, disposable sterile syringes are mainly used for medication injection, but to perform the injection process, it is required to see a doctor, and the operation and injection are performed by professionals, it's not convenient to use, and is not suitable for self-management of most patients with chronic disease. Large companies at home and abroad mostly concentrate on fields and directions such as needle-free injection, automatic injection, electronic injection pens and purely mechanical injection pens and the like, however, these solutions generally have obvious disadvantages such as low reliability, high cost, and may even cause skin damage with long-term use. Currently, purely mechanical injection pens have the best safety and reliability performance in the structures of screw transmission, clutch transmission and tactile feedback during the accurate dispensing process of medication. However, most of the conventional mechanical injection pens have the defects such as cumbersome steps in the injection process and large resistance, which are inconvenient for the patients to use, and due to many components and complicated structure of the conventional mechanical injection pen, there is a high probability that a deviation of injection dose may occur. Besides, since most patients are elderly and have mobility difficulties, easy-to-identify measurement display structures and prompts are required.

Therefore, a technical issue to be addressed by those skilled in the art is to provide an injection pen, which is easy to use and the injection dose thereof can be accurately controlled.

### SUMMARY

An object of the present application to provide an injection pen, which is easy to use and the injection dose thereof can be accurately controlled.

In order to address the above technical issue, an injection pen is provided according to the present application, including a pen tube. A medication cartridge is arranged at a front end portion of the pen tube, and a trigger is arranged at a rear end portion of the pen tube. A dosage tube, a differential rod and a drive rod are coaxially arranged inside the pen tube in sequence from outside to inside, and the drive rod is connected to the differential rod through a key-and-groove connection. An inner wall of the pen tube is provided with a position-limiting protrusion cooperating with the drive rod. A drive clutch is arranged at a front end portion of the dosage tube, a rear end portion of the drive clutch is provided with a clamping tooth, the drive rod is provided with an inner ring tooth engaged with the clamping tooth, and the drive clutch is coupled with the dosage tube in an unidirectionally drivable manner. The front end portion of the dosage tube is further provided with a push rod seat, and a front portion of the push rod seat is provided with a push sheet. A push rod cooperating with the push sheet is coaxially arranged inside the push rod seat through threaded engagement, and the push rod seat is coupled with the drive rod in the unidirectionally drivable manner.

Preferably, a position-limiting ring in threaded engagement with the differential rod is clamp-fitted on the dosage tube.

Preferably, the differential rod is provided with a ribbed plate, and the rear end portion of the drive clutch is provided with a flexible structural member in contact with and cooperating with the ribbed plate.

Preferably, the push rod seat is provided with a ratchet wheel, and the drive rod is provided with multiple first one-way ratchets engaged with the ratchet wheel.

Preferably, each of an outer wall of a front end portion of the drive clutch and an inner wall of the front end portion of the dosage tube is provided with second one-way ratchets that are engaged with each other.

Preferably, a drive sleeve cooperating with the drive clutch is arranged at the front end portion of the dosage tube, and each of the dosage tube and the drive clutch is coupled with the drive sleeve in the unidirectionally drivable manner.

Preferably, the rear end portion of the pen tube is provided with a bracket coupled with the dosage tube, and the trigger is arranged on the bracket.

Preferably, the trigger is a button.

Preferably, a pen cap cooperating with the medication cartridge is detachably arranged at the front end portion of the pen tube.

Compared with the background technology, during use, the injection pen according to the present application allows the dosage tube and the pen tube to move toward an end where the trigger is located by forward rotation of the dosage tube and driving cooperation between the drive members such as the drive clutch, the differential rod and the drive rod and the like, thereby achieving the setting of the medication dosage before injection. During the subsequent operation, the dosage tube rotates backward, and due to the one-way drive mechanisms, the dosage tube is allowed to move toward an end where the medication cartridge is located, thereby completing the reverse adjustment of the medication dosage. During injection, the trigger is pressed, the dosage tube is coupled to the trigger and cooperates with the trigger, and the dosage tube moves toward the end where the medication cartridge is located and rotates backward. At the same time, the push rod is allowed to rotate in the same direction as the dosage tube due to the one-way drive mechanisms. While the push rod is rotating, the push rod is allowed to move axially through the threaded engagement structure between the inner wall of the push rod seat and the outer wall of the push rod, which drives the push sheet to move toward the end where the medication cartridge is located, thereby completing the injection operation. After the injection is completed, the fitting members such as the dosage tube, the differential rod, the trigger and the like are returned to the pen tube. The operation process of the injection pen is simple and easy, the operation resistance is small, the drive components are flexible and reliable, the control accuracy of the medication dosage is high, and the usage of medication is accurate and controllable.

### BRIEF DESCRIPTION OF THE DRAWINGS

For more clearly illustrating embodiments of the present application or the technical solutions in the conventional technology, drawings referred to describe the embodiments or the conventional technology will be briefly described hereinafter. Apparently, the drawings in the following description are only some examples of the present application, and for those skilled in the art, other drawings may be obtained based on these drawings without any creative efforts.
Figure 1 is an exploded view showing the structure of an injection pen according to a specific embodiment of the present application;
Figure 2 is a schematic view showing an assembly of the structure of a main body in Figure 1;
Figure 3 is a sectional structural view taken in a direction A-A in Figure 2;
Figure 4 is a sectional structural view taken in a direction B-B in Figure 2;
Figure 5 is a sectional structural view taken in a direction C-C in Figure 2;
Figure 6 is a sectional structural view taken in a direction D-D in Figure 2;
Figure 7 is a sectional structural view taken in a direction E-E in Figure 2; and
Figure 8 is an exploded view showing the structure of an injection pen according to another specific embodiment of the present application.

### DETAILED DESCRIPTION OF THE EMBODIMENTS

An object of the present application is to provide an injection pen which is easy to use, and a medication dosage is accurate and controllable.

For those skilled in the art to better understand technical solutions of the present application, the present application will be further described in detail in conjunction with drawings and embodiments hereinafter.

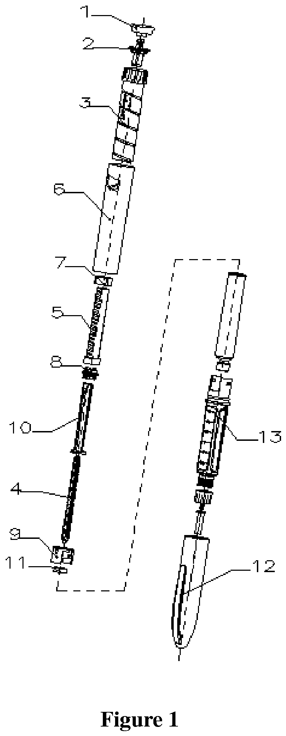

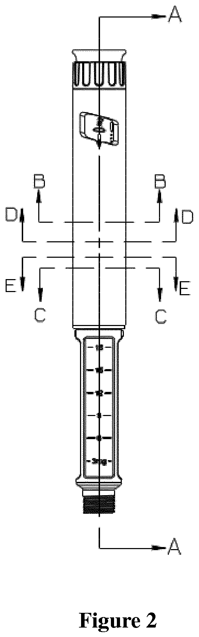

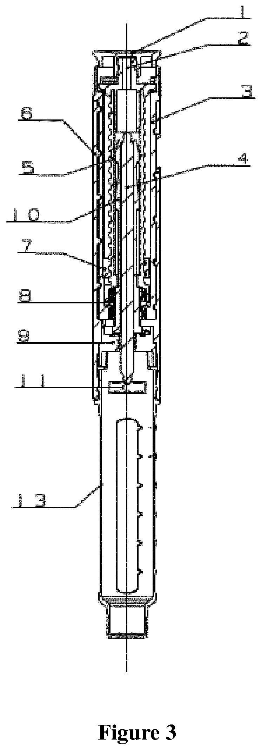

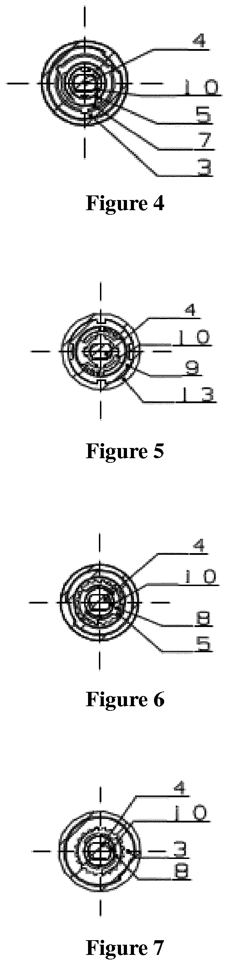

Referring to Figures 1 to 7, Figure 1 is an exploded view showing the structure of an injection pen according to a specific embodiment of the present application; Figure 2 is a schematic view showing an assembly of the structure of a main body in Figure 1; Figure 3 is a sectional structural view taken in a direction A-A in Figure 2; Figure 4 is a sectional structural view taken in a direction B-B in Figure 2; Figure 5 is a sectional structural view taken in a direction C-C in Figure 2; Figure 6 is a sectional structural view taken in a direction D-D in Figure 2; and Figure 7 is a sectional structural view taken in a direction E-E in Figure 2.

In a specific embodiment, the injection pen according to the present application includes a pen tube 6. A medication cartridge 13 is arranged at a front end portion of the pen tube 6, and a trigger 1 is arranged at a rear end portion of the pen tube 6. A dosage tube 3, a differential rod 5 and a drive rod 10 are coaxially arranged inside the pen tube 6 in sequence from outside to inside, and the drive rod 10 is connected to the differential rod 5 through a key-and-groove connection. An inner wall of the pen tube 6 is provided with a position-limiting protrusion cooperating with the drive rod 10. A drive clutch 8 is arranged at a front end portion of the dosage tube 3, a rear end portion of the drive clutch 8 is provided with a clamping tooth (not shown in the drawings), the drive rod 5 is provided with an inner ring tooth (not shown in the drawings) engaged with the clamping tooth, and the drive clutch 8 is coupled with the dosage tube 3 in an unidirectionally drivable manner. The front end portion of the dosage tube 3 is further provided with a push rod seat 9, and a front portion of the push rod seat 9 is provided with a push sheet 11. A push rod 4 cooperating with the push sheet 11 is coaxially arranged inside the push rod seat 9 through threaded engagement, and the push rod seat 9 is coupled with the drive rod 10 in the unidirectionally drivable manner.

During an operation process, through forward rotation of the dosage tube 3 and driving cooperation between drive members such as the drive clutch 8, the differential rod 5, the drive rod 10 and the like, the dosage tube 3 and the pen tube 6 are allowed to move toward an end where the trigger 1 is located, thereby achieving setting of the medication dosage before injection. During subsequent operation, the dosage tube 3 rotates backward, and due to one-way drive mechanisms, the dosage tube 3 is allowed to move toward an end where the medication cartridge 13 is located, thereby completing reverse adjustment of the medication dosage. During injection, the trigger 1 is pressed, the dosage tube 3 is coupled to the trigger 1 and cooperates with the trigger, and the dosage tube 3 moves toward the end where the medication cartridge 13 is located and rotates backward. At the same time, the push rod 4 is allowed to rotate in a same direction as the dosage tube 3 due to the one-way drive mechanisms. While the push rod 4 is rotating, the push rod 4 is allowed to move axially through a threaded engagement structure between an inner wall of the push rod seat 9 and an outer wall of the push rod 4, thus the push sheet 11 is driven to move toward the end where the medication cartridge 13 is located, thereby completing the injection. After the injection is completed, fitting members such as the dosage tube 3, the differential rod 5, the trigger 1 and the like are returned to the pen tube 6. The operation process of the injection pen is simple and easy, an operation resistance is small, drive components are flexible and reliable, control accuracy of the medication dosage is high, and usage of medication is accurate and controllable.

Furthermore, a position-limiting ring 7 in threaded engagement with the differential rod 5 is clamp-fitted on the dosage tube 3. In actual operation, the dosage tube 3 and the position-limiting ring 7 are clamp-fitted to each other and cannot rotate with respect to each other, instead, the dosage tube 3 and the position-limiting ring 7 can only move with respect to each other in an axial direction. Realization of a limit function of the position-limiting ring 7 in three processes is described as follows.
a. During a dosage setting process, the dosage tube 3 rotates forward and drives the position-limiting ring 7 to rotate forward, the differential rod 5 does not rotate, and the position-limiting ring 7 is in threaded engagement with the differential rod 5, thereby generating a backward relative displacement;
b. During a reverse dosage adjustment process, the dosage tube 3 rotates backward and drives the position-limiting ring 7 to rotate backward and move forward, the differential rod 5 does not rotate, and the position-limiting ring 7 rotates with respect to the differential rod 5, thereby generating a forward axial displacement; and
c. During an injection process, both the dosage tube 3 and the differential rod 5 rotate backward and move forward synchronously in the axial direction, the position-limiting ring 7 does not rotate with respect to the dosage tube 3, and does not rotate with respect to the differential rod 5, and there is no axial displacement generated between the position-limiting ring 7 and the differential rod 5.

During the dosage adjustment process, a backward displacement of the position-limiting ring 7 with respect to the differential rod 5 is generated according to the adjusted dosage. During the injection process, there is no axial displacement generated between the position-limiting ring 7 and the differential rod 5. After each time of injection, the position-limiting ring 7 is always located further rearward with respect to the differential rod 5 than after a previous time of injection. After pre-filled medication is consumed out, the position-limiting ring 7 reaches a rearmost end of the differential rod 5 and is clamped by a left end of a bracket 2, and can no longer be used, thereby realizing disposable use of the injection pen.

More specifically, the differential rod 5 is provided with a ribbed plate, and a rear end portion of the drive clutch 8 is provided with a flexible structural member in contact with and cooperating with the ribbed plate. During the operation, the flexible structural member can come into contact with and cooperate with the ribbed plate to make a special sound, thereby forming an auditory prompt to the operator, and realizing a non-visual prompt during the setting process of medication dosage, and further improving the convenience of using the injection pen.

Besides, the push rod seat 9 is provided with a ratchet wheel, and the drive rod 10 is provided with a plurality of first one-way ratchets engaged with the ratchet wheel. Driving of an one-way drive mechanism composed of the ratchet wheel and the first one-way ratchets is stable and reliable, and is helpful to improve driving efficiency and operation accuracy between the push rod seat 9 and the drive rod 10.

Moreover, each of an outer wall of a front end portion of the drive clutch 8 and an inner wall of the front end portion of the dosage tube 3 is provided with second one-way ratchets that are engaged with each other. A drive mechanism formed directly by engagement of the second one-way ratchets helps to simplify an overall number of components of the injection pen, and optimize its assembly structure, and thereby reducing an equipment cost correspondingly.

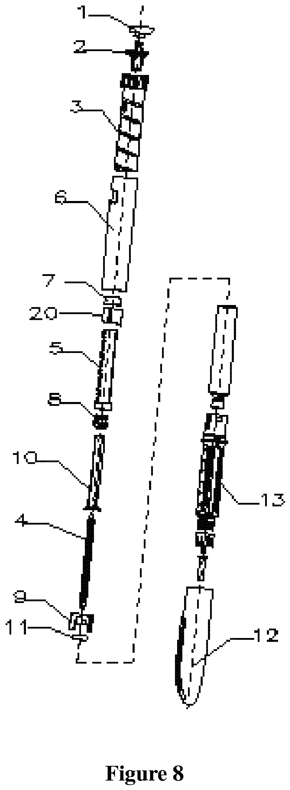

Referring to Figure 8, Figure 8 is an exploded view showing the structure of an injection pen according to another specific embodiment of the present application.

In addition, a drive sleeve 20 cooperating with the drive clutch 8 is arranged at the front end portion of the dosage tube 3, and each of the dosage tube 3 and the drive clutch 8 is coupled with the drive sleeve 20 in an unidirectional drivable manner. By additionally providing the drive sleeve 20, the drive mechanism between the dosage tube 3 and the drive clutch 8 can be further optimized, which ensures control accuracy and stability of the driving between the dosage tube 3 and the drive clutch 8.

It should be noted that, in practical use, cooperating structures between the dosage tube 3, the drive sleeve 20 and the drive clutch 8 may employ the one-way ratchet wheel engagement mechanism as described above or other mechanical structures capable of realizing one-way driving. In principle, the cooperating structures may employ any structure as long as it can meet requirements of actual use of the injection pen.

Continued reference is made to Figures 1 to 7. The rear end portion of the pen tube 6 is provided with a bracket 2 coupled with the dosage tube 3, and the trigger 1 is arranged on the bracket 2. The bracket 2 can provide certain structural support for the trigger 1 and ensure the driving efficiency and structural reliability between the trigger 1 and the dosage tube 3.

Further, the trigger 1 is a button. The button has a simple and ingenious structure, which effectively optimizes an overall operation structure of the injection pen. Obviously, it is just a preferable solution that the trigger 1 is a button. In practice, the trigger may be other operating members such as a push rod or the like. In principle, the trigger may be any operating member as long as it can meet the requirements of actual use of the injection pen.

More specifically, a pen cap 12 cooperating with the medication cartridge 13 is detachably arranged at the front end portion of the pen tube 6. The pen cap 12 can provide reliable structural protection for the medication cartridge 13 and a main structure located at a front portion of the pen tube 6, to avoid structural damages of main functional components of the injection pen, and avoid contamination or other adverse effects of internal components of the injection pen caused by an external environment in a non-use state.

In order to facilitate understanding of the solution, the present application is appropriately illustrated hereinafter in conjunction with an actual operation process.

During the dosage setting process, the dosage tube 3 rotates forward, one-way ratchets of the dosage tube 3 acts on the second one-way ratchets located at the front end portion of the drive clutch 8, and the drive clutch 8 does not rotate and generates an axial displacement toward the end where the trigger is located (that is a rear end), the drive clutch 8 moves backward in the axial direction, to push the differential rod 5, the bracket 2 and the trigger 1 to move backward in the axial direction. The drive rod 10 is connected to the differential rod 5 through a key-and-groove connection, but the push rod seat 9 is clamped by a protrusion at the inner wall of the pen tube 6 and cannot move backward, a flexible ratchet wheel at a front end of the drive rod 10 is fixed with respect to the push rod seat 9, therefore the flexible ratchet cannot move back and forth; the rear end portion of the drive clutch 8 is provided with the flexible structural member which cannot drive the differential rod 5 to rotate, since the differential rod 5 does not rotate, the drive clutch 8 does not rotate, and the second one-way ratchets of the drive clutch 8 interact with the one-way ratchets of the dosage tube 3 to generate an auditory prompt, at the same time, the dosage tube 3, the trigger 1, the bracket 2 and the differential rod 5 are pushed toward the rear end to get out of the pen tube 6, thereby completing the dosage setting.

During the reverse dosage adjustment process, the dosage tube 3 rotates backward, the one-way ratchets of the dosage tube 3 drives the drive clutch 8 to rotate, the medication cartridge provides the push sheet 11 and the push rod 4 with a reaction force to counteract a dosage that is to be adjusted reversely, a reverse rotational friction is generated between the flexible structural member at the rear end portion of the drive clutch 8 and the ribbed plate of the differential rod 5. The drive rod 10 and the differential rod 5 are connected through the key-and-groove connection and do not rotate with respect to each other, the drive rod 10 does not rotate, the push rod seat 9 does not rotate, and an axial forward displacement of the push rod 4 which has been generated is completely counteracted. The dosage tube 3 moves forward in the axial direction, and drives the trigger 1, the bracket 2, and the differential rod 5 to move forward, thereby realizing the reverse adjustment of the dosage. The drive clutch 8 rotates, and cooperates with the ribbed plate of the differential rod 5 to generate the auditory prompt.

During the injection process, the trigger 1 is pressed, the dosage tube 3 is pressed to move forward in the axial direction and rotate backward, the one-way ratchets of the dosage tube 3 drives the drive clutch 8 to rotate, in this case, one-way ratchets of the drive clutch 8 engaged with the differential rod 5 cannot perform driving. However, with the pressing, four projecting ratchets on a top surface of the drive clutch 8 are engaged with a circle of ratchets inside the differential rod 5, so that the dosage tube 3 can drive the drive clutch 8 to rotate backward and further drive the differential rod 5 to rotate backward, causing the drive rod 10 to rotate. The ratchet wheel of the drive rod 10 is engaged with the one-way ratchets of the push rod seat 9, and the push rod 4 also rotates backward, at the same time, the threaded engagement structure between the inner wall of the push rod seat 9 and the outer wall of the push rod 4 drives the push rod 4 to move forward in the axial direction, to press against the push sheet 11 for injection. After the injection is completed, the dosage tube 3, the trigger 1, the bracket 2, and the differential rod 5 are returned to the pen tube 6 together.

As described above, the injection pen according to the present application includes the pen tube. The medication cartridge is arranged at the front end portion of the pen tube, and the trigger is arranged at the rear end portion of the pen tube. The dosage tube, the differential rod and the drive rod are coaxially arranged inside the pen tube in sequence from outside to inside, and the drive rod is connected to the differential rod through the key-and-groove connection. The inner wall of the pen tube is provided with the position-limiting protrusion cooperating with the drive rod. The drive clutch is arranged at the front end portion of the dosage tube, the rear end portion of the drive clutch is provided with the clamping tooth, the drive rod is provided with the inner ring tooth engaged with the clamping tooth, and the drive clutch is coupled with the dosage tube in the unidirectionally drivable manner. The front end portion of the dosage tube is further provided with the push rod seat, and the front portion of the push rod seat is provided with the push sheet. The push rod cooperating with the push sheet is coaxially arranged inside the push rod seat through threaded engagement, and the push rod seat is coupled with the drive rod in the unidirectionally drivable manner. During the operation process, through forward rotation of the dosage tube and driving cooperation between drive members such as the drive clutch, the differential rod, the drive rod and the like, the dosage tube and the pen tube are allowed to move toward the end where the trigger is located, thereby achieving setting of the medication dosage before injection. During subsequent operation, the dosage tube rotates backward, and due to one-way drive mechanisms, the dosage tube is allowed to move toward the end where the medication cartridge is located, thereby completing reverse adjustment of the medication dosage. During injection, the trigger is pressed, the dosage tube is coupled to the trigger and cooperates with the trigger, and the dosage tube moves toward the end where the medication cartridge is located and rotates backward. At the same time, the push rod is allowed to rotate in the same direction as the dosage tube due to the one-way drive mechanisms. While the push rod is rotating, the push rod is allowed to move axially through the threaded engagement structure between the inner wall of the push rod seat and the outer wall of the push rod, thus the push sheet is driven to move toward the end where the medication cartridge is located, thereby completing the injection. After the injection is completed, the fitting members such as the dosage tube, the differential rod, the trigger and the like are returned to the pen tube. The operation process of the injection pen is simple and easy, the operation resistance is small, the drive components are flexible and reliable, the control accuracy of the medication dosage is high, and the usage of medication is accurate and controllable.

The injection pen according to the present application is described in detail hereinbefore. The principle and the embodiments of the present application are illustrated herein by specific examples. The above description of examples is only intended to help the understanding of the method and the spirit of the present application. It should be noted that, for those skilled in the art, a few of modifications and improvements may be made to the present application without departing from the principle of the present application, and these modifications and improvements are also deemed to fall into the scope of the present application defined by the claims.

## Claims

1. An injection pen, comprising a pen tube, wherein
a medication cartridge is arranged at a front end portion of the pen tube, a trigger is arranged at a rear end portion of the pen tube, and a dosage tube, a differential rod and a drive rod are coaxially arranged inside the pen tube in sequence from outside to inside;
the drive rod is connected to the differential rod through a key-and-groove connection; an inner wall of the pen tube is provided with a position-limiting protrusion cooperating with the drive rod, a drive clutch is arranged at a front end portion of the dosage tube, a rear end portion of the drive clutch is provided with a clamping tooth, the drive rod is provided with an inner ring tooth engaged with the clamping tooth, and the drive clutch is coupled with the dosage tube; and
the front end portion of the dosage tube is further provided with a push rod seat, and a front portion of the push rod seat is provided with a push sheet, a push rod cooperating with the push sheet is coaxially arranged inside the push rod seat through threaded engagement, and the push rod seat is coupled with the drive rod.

2. The injection pen according to claim 1, wherein a position-limiting ring in threaded engagement with the differential rod is clamp-fitted on the dosage tube.

3. The injection pen according to claim 1, wherein the differential rod is provided with a ribbed plate, and the rear end portion of the drive clutch is provided with a flexible structural member in contact with and cooperating with the ribbed plate.

4. The injection pen according to claim 1, wherein the push rod seat is provided with a ratchet wheel, and the drive rod is provided with a plurality of first one-way ratchets engaged with the ratchet wheel.

5. The injection pen according to claim 1, wherein each of an outer wall of a front end portion of the drive clutch and an inner wall of the front end portion of the dosage tube is provided with second one-way ratchets that are engaged with each other.

6. The injection pen according to claim 1, wherein a drive sleeve cooperating with the drive clutch is arranged at the front end portion of the dosage tube, and each of the dosage tube and the drive clutch is coupled with the drive sleeve in an unidirectionally drivable manner.

7. The injection pen according to claim 1, wherein the rear end portion of the pen tube is provided with a bracket coupled with the dosage tube, and the trigger is arranged on the bracket.

8. The injection pen according to claim 1, wherein the trigger is a button.

9. The injection pen according to claim 1, wherein a pen cap cooperating with the medication cartridge is detachably arranged at the front end portion of the pen tube.
